# EUROPEAN PATENT APPLICATION

(11) **EP 4 736 879 A1**
(43) Date of publication of application: **06.05.2026**
(21) Application number: 24918323.7
(22) Date of filing: 20.12.2024
(51) Int. Cl.: A61K 47/24, A61K 47/26, A61K 9/08, A61K 31/445, A61K 31/5415, A61K 31/635, A61P 29/00, A61K 47/10, A61K 47/14, A61K 47/22

(54) **SUSTAINED-RELEASE ANALGESIC PHARMACEUTICAL COMPOSITION, METHOD FOR PREPARING SAME, AND USE THEREOF**

(30) Priority: 19.01.2024 CN 202410080358
(71) Applicant: Brightintel Biotech Pharmaceutical Co., Ltd., Suzhou, Jiangsu 215000 (CN)
(72) Inventor: LI, Fengquan, Suzhou, Jiangsu 215000 (CN); YE, Qiongru, Suzhou, Jiangsu 215000 (CN); LI, Jianxuan, Suzhou, Jiangsu 215000 (CN)
(74) Representative: Purdylucey Intellectual Property
(86) International application number: PCT/CN2024/141226
(87) International publication number: WO 2025/152715

(57) **Abstract**

The present application belongs to the field of pharmaceutical preparations, and particularly relates to a sustained-release analgesic pharmaceutical composition, a method for preparing same, and use thereof. The pharmaceutical composition of the present application comprises a drug, a delivery carrier, and a release regulator. The drug is selected from at least one of a local anesthetic and a non-steroidal anti-inflammatory drug. The release regulator is selected from a phospholipid compound. The delivery carrier is selected from a saccharide and an esterified form thereof. According to the present application, the coaction of the phospholipid compound and the delivery carrier can increase the plasma drug concentration upon the initial release in vivo, reduce the time to peak, enhance the analgesic effect in the early stage after a surgery, and ensure the long-term release of the drug, thus effectively relieving the postoperative pain, reducing the administration frequency, and promoting skin wound healing. The use of a specific solubilizer provides good stability for the drug and prevents precipitation. The composition can be administered through the wound, making it easy to use.

## Description

### TECHNICAL FIELD

The present disclosure belongs to the technical field of pharmaceutical preparations, and in particular relates to a sustained-release analgesic pharmaceutical composition, a method for preparing the same and use thereof.

### BACKGROUND

Pain is one of the most common postoperative complications. Currently, one or more drugs among opioid analgesics, non-steroidal anti-inflammatory drugs, and local anesthetics are mainly used for postoperative analgesia in clinical practice. Among them, local anesthetics such as Bupivacaine Hydrochloride Injection and Ropivacaine Hydrochloride Injection have a short duration of action; the analgesic effect of a single dose lasts only 6-8 hours, which cannot meet the treatment cycle of postoperative pain and is difficult to relieve the pain of patients during postoperative treatment for a long time. Meanwhile, there is no sustained-release and long-acting dosage form of anti-inflammatory drugs yet. For example, Anjeso Injection (US11253478B2) is a daily dosage form, which is difficult to meet the requirement of long-term sustained release of drug efficacy for multiple days. Therefore, opioid drugs commonly used clinically or those administered continuously with analgesic pumps not only have poor clinical compliance but also cause many side effects, affecting patients' prognosis.

To solve the above problems, the foreign marketed drug Posimir (Publication No.: CN101035562A) uses sucrose acetate isobutyrate as a sustained-release carrier and benzyl alcohol as a solvent, with a bupivacaine concentration of 132 mg/mL and a benzyl alcohol dosage of approximately 22% w/w. Due to the certain neurotoxicity of benzyl alcohol, there may be safety issues when the dosage is large; at the same time, the drug has insufficient efficacy and many adverse reactions at the wound site. The foreign marketed drug Zynrelef (Publication No.: CN115025099A) is a compound preparation of bupivacaine and meloxicam, using polyorthoester polymer as the sustained-release carrier. After being administered to the administration site, it achieves a sustained-release effect through the slow degradation of the polyorthoester itself. During the prescription development, precipitation of bupivacaine is observed, and subsequently, maleic acid is added to the prescription to prevent precipitation of raw materials. However, maleic acid is acidic, which can cause adverse reactions at the wound and affect wound healing. Meanwhile, its drug release rate is slow and the time to peak concentration is long, so it cannot exert the required analgesic effect when patients experience severe pain in the early postoperative period.

Therefore, there is an urgent need to provide a sustained-release analgesic pharmaceutical composition that can increase the initial release concentration of the drug in the body, enhance the early analgesic effect, maintain sustained release, and promote good wound healing.

### SUMMARY

The present disclosure aims to solve one or more technical problems existing in the existing technologies, and at least provide a beneficial option or create conditions. Specifically, the present disclosure provides a sustained-release analgesic pharmaceutical composition that can increase the initial release concentration of the drug in vivo, enhance the early analgesic efficacy while maintaining sustained release, and promote good wound healing.

The inventive concept of the present disclosure is as follows: the sustained-release analgesic pharmaceutical composition of the present disclosure includes a drug, a delivery carrier, and a release-regulating agent; the drug is at least one selected from the group consisting of a local anesthetic and a nonsteroidal anti-inflammatory drug; the release-regulating agent is selected from a phospholipid compound; the delivery carrier is selected from a saccharide and an esterified form thereof.

The present disclosure uses a phospholipid compound as the release-regulating agent, which, together with the delivery carrier, can increase the in vivo plasma concentration upon the initial release, enhance the early analgesic efficacy and maintain sustained release. It is exactly opposite to the existing technologies that delays the sustained release duration of the active pharmaceutical ingredient and has a longer time to reach peak drug release. Furthermore, the pharmaceutical composition of the present disclosure can promote wound healing.

Therefore, in a first aspect, the present disclosure provides a sustained-release analgesic pharmaceutical composition.

Specifically, the sustained-release analgesic pharmaceutical composition includes a drug, a delivery carrier, and a release-regulating agent;
wherein the drug is at least one selected from the group consisting of a local anesthetic and a nonsteroidal anti-inflammatory drug;
wherein the release-regulating agent is selected from a phospholipid compound; and
wherein the delivery carrier is selected from a saccharide and an esterified form thereof.

Specifically, the inventors added phospholipids to the pharmaceutical composition to explore their effects on skin wound healing and repair. Surprisingly, in the sustained-release system of the present disclosure, the addition of phospholipids can increase the in vivo plasma concentration upon the initial release without causing obvious burst release, thereby enhancing the early analgesic efficacy, effectively alleviating patients' severe pain in the early postoperative period and maintaining sustained drug release, achieving unexpected technical effects. In general, phospholipids are added to many drugs as excipients to delay release, such as the in-situ gel system disclosed in Publication No. CN103705442A. The aforementioned beneficial effects of the present disclosure are completely opposite to the technical effect of the existing technologies where the addition of a release-regulating agent only delays the sustained release duration of the active pharmaceutical ingredient.

Preferably, when the drug contains a local anesthetic, the pharmaceutical composition further contains a solubilizer.

Preferably, the solubilizer is at least one selected from the group consisting of menthol, camphor, borneol, linalool, and eucalyptol.

Specifically, the addition of a solubilizer to the pharmaceutical composition of the present disclosure can increase the solubility of the active pharmaceutical ingredient. The solubilizer works together with other components to ensure that the prepared product does not precipitate the drug during the preparation process and during sustained low-temperature storage, exhibiting good stability. Meanwhile, by selecting specific types of solubilizers, the adverse effects of organic acids or inorganic acids on the wound can be avoided, without reducing the pH value of the wound or causing adverse impacts on wound inflammation and healing.

Preferably, the local anesthetic is selected from an amide anesthetic.

Preferably, the amide anesthetic is at least one selected from the group consisting of bupivacaine, ropivacaine, and a pharmaceutically acceptable salt or a stereoisomer thereof.

Preferably, the nonsteroidal anti-inflammatory drug is at least one selected from the group consisting of meloxicam, celecoxib, and a pharmaceutically acceptable salt or a stereoisomer thereof.

Preferably, the delivery carrier is at least one selected from the group consisting of sucrose, chitosan, sucrose acetate isobutyrate, sucrose octaacetate, and sucrose monoacetate monoisobutyrate.

Preferably, the phospholipid compound is at least one selected from the group consisting of a natural phospholipid and a synthetic phospholipid.

Preferably, the natural phospholipid is at least one selected from the group consisting of a soybean phosphatidylcholine (SPC), an egg yolk phosphatidylcholine (EPC), a rapeseed phospholipid, and a sunflower phospholipid.

Preferably, the synthetic phospholipid is at least one selected from the group consisting of dierucoyl phosphatidylcholine (DEPC), dioleoyl phosphatidylcholine (DOPC), palmitoyl oleoyl phosphatidylcholine (POPC), distearoyl phosphatidylcholine (DSPC), dipalmitoyl phosphatidylglycerol (DPPG), and distearoyl phosphatidylglycerol (DSPG).

Preferably, the mass content of phosphatidylcholine (PC) in the SPC is 60% to 99%.

Preferably, the menthol is L-menthol.

Preferably, after administration of the pharmaceutical composition to an animal, when the drug contains the local anesthetic, the time to peak plasma concentration of the local anesthetic is 0.1 h to 8 h; when the drug is selected from a nonsteroidal anti-inflammatory drug, the time to peak plasma concentration is 1 h to 28 h.

More preferably, after in vivo administration of the pharmaceutical composition to an animal, when the drug contains the local anesthetic, the time to peak plasma concentration of the local anesthetic is 0.4 h to 4 h; when the drug is selected from a nonsteroidal anti-inflammatory drug, the time to peak plasma concentration is 2 h to 10 h.

Preferably, after administration of the pharmaceutical composition to a SD rat by injection, when the drug contains the local anesthetic, the time to peak plasma concentration of the local anesthetic is 0.2 h to 4 h; when the drug is selected from a nonsteroidal anti-inflammatory drug, the time to peak plasma concentration is 4 h to 24 h.

More preferably, after administration of the pharmaceutical composition to a SD rat by injection, when the drug contains the local anesthetic, the time to peak plasma concentration of the local anesthetic is 0.5 h to 2 h; when the drug is selected from a nonsteroidal anti-inflammatory drug, the time to peak plasma concentration is 8 h to 20 h.

Preferably, after administration of the pharmaceutical composition to a beagle dog by injection, when the drug contains the local anesthetic, the time to peak plasma concentration of the local anesthetic is 1 h to 4 h; when the drug is selected from a nonsteroidal anti-inflammatory drug, the time to peak plasma concentration is 4 h to 12 h.

More preferably, after administration of the pharmaceutical composition to a beagle dog by injection, when the drug contains the local anesthetic, the time to peak plasma concentration of the local anesthetic is 2 h to 3 h; when the drug is selected from a nonsteroidal anti-inflammatory drug, the time to peak plasma concentration is 4 h to 8 h.

Preferably, the sustained-release analgesic pharmaceutical composition includes 0.01-20 parts by weight of the drug, 30-80 parts by weight of the delivery carrier, 0.05-20 parts by weight of the release-regulating agent.

More preferably, the sustained-release analgesic pharmaceutical composition includes 0.01-8 parts by weight of the drug, 50-75 parts by weight of the delivery carrier, 0.05-10 parts by weight of the release-regulating agent.

Further preferably, the sustained-release analgesic pharmaceutical composition includes 0.01-6 parts by weight of the drug, 55-68 parts by weight of the delivery carrier, 0.05-5 parts by weight of the release-regulating agent.

Preferably, when the drug contains the local anesthetic, the sustained-release analgesic pharmaceutical composition includes 0.01-20 parts by weight of the drug, 30-80 parts by weight of the delivery carrier, more than 0 and less than or equal to 10 parts by weight of the solubilizer, 0.05-20 parts by weight of the release-regulating agent.

More preferably, when the drug contains the local anesthetic, the sustained-release analgesic pharmaceutical composition includes 1-8 parts by weight of the drug, 50-70 parts by weight of the delivery carrier, more than 0 and less than or equal to 5 parts by weight of the solubilizer, 0.05-10 parts by weight of the release-regulating agent.

Further preferably, when the drug contains a local anesthetic, the sustained-release analgesic pharmaceutical composition includes 2-6 parts by weight of the drug, 55-68 parts by weight of the delivery carrier, more than 0 and less than or equal to 3 parts by weight of the solubilizer, 0.05-5 parts by weight of the release-regulating agent.

Preferably, when the drug is selected from a nonsteroidal anti-inflammatory drug, the sustained-release analgesic pharmaceutical composition includes 0.1-20 parts by weight of the drug, 30-80 parts by weight of the delivery carrier, 0-10 parts by weight of the solubilizer, 0.05-20 parts by weight of the release-regulating agent.

More preferably, when the drug is selected from a nonsteroidal anti-inflammatory drug, the sustained-release analgesic pharmaceutical composition includes 0.5-10 parts by weight of the drug, 50-70 parts by weight of the delivery carrier, 0-5 parts by weight of the solubilizer, 0.05-10 parts by weight of the release-regulating agent.

Further preferably, when the drug is selected from a nonsteroidal anti-inflammatory drug, the sustained-release analgesic pharmaceutical composition includes 1-5 parts by weight of the drug, 55-68 parts by weight of the delivery carrier, 0-3 parts by weight of the solubilizer, 0.05-5 parts by weight of the release-regulating agent.

Preferably, when the drug is selected from a local anesthetic and a nonsteroidal anti-inflammatory drug, the sustained-release analgesic pharmaceutical composition includes 0.01-15 parts by weight of the local anesthetic, 0.01-5 parts by weight of the nonsteroidal anti-inflammatory drug, 30-80 parts by weight of the delivery carrier, more than 0 and less than or equal to 10 parts by weight of the solubilizer, and 0.05-20 parts by weight of the release-regulating agent.

More preferably, when the drug is selected from a local anesthetic and a nonsteroidal anti-inflammatory drug, the sustained-release analgesic pharmaceutical composition includes 1-5 parts by weight of the local anesthetic, 0.01-3 parts by weight of the nonsteroidal anti-inflammatory drug, 50-70 parts by weight of the delivery carrier, more than 0 and less than or equal to 5 parts by weight of the solubilizer, and 0.05-10 parts by weight of the release-regulating agent.

Further preferably, when the drug is selected from a local anesthetic and a nonsteroidal anti-inflammatory drug, the sustained-release analgesic pharmaceutical composition includes 2-4 parts by weight of the local anesthetic, 0.01-2 parts by weight of the nonsteroidal anti-inflammatory drug, 55-68 parts by weight of the delivery carrier, more than 0 and less than or equal to 3 parts by weight of the solubilizer, and 0.05-5 parts by weight of the release-regulating agent.

Preferably, the sustained-release analgesic pharmaceutical composition further includes a solvent; or the sustained-release analgesic pharmaceutical composition further includes a solvent and an antioxidant.

Preferably, when the drug contains the local anesthetic, the sustained-release analgesic pharmaceutical composition includes 0.01-20 parts by weight of the drug, 30-80 parts by weight of the delivery carrier, more than 0 and less than or equal to 10 parts by weight of the solubilizer, 0.05-20 parts by weight of the release-regulating agent, more than 0 and less than or equal to 70 parts by weight of the solvent, and 0-5 parts by weight of the antioxidant; when the drug is selected from a nonsteroidal anti-inflammatory drug, the sustained-release analgesic pharmaceutical composition includes 0.1-20 parts by weight of the drug, 30-80 parts by weight of the delivery carrier, 0-10 parts by weight of the solubilizer, 0.05-20 parts by weight of the release-regulating agent, more than 0 and less than or equal to 70 parts by weight of the solvent, and 0-5 parts by weight of the antioxidant.

More preferably, when the drug contains a local anesthetic, the sustained-release analgesic pharmaceutical composition includes 1-8 parts by weight of the drug, 50-70 parts by weight of the delivery carrier, more than 0 and less than or equal to 5 parts by weight of the solubilizer, 0.05-10 parts by weight of the release-regulating agent, 20-60 parts by weight of the solvent, and 0-2 parts by weight of the antioxidant; when the drug is selected from a nonsteroidal anti-inflammatory drug, the sustained-release analgesic pharmaceutical composition includes 0.5-10 parts by weight of the drug, 50-70 parts by weight of the delivery carrier, 0-5 parts by weight of the solubilizer, 0.05-10 parts by weight of the release-regulating agent, 20-60 parts by weight of the solvent, and 0-2 parts by weight of the antioxidant.

Further preferably, when the drug contains a local anesthetic, the sustained-release analgesic pharmaceutical composition includes 2-6 parts by weight of the drug, 55-68 parts by weight of the delivery carrier, more than 0 and less than or equal to 3 parts by weight of the solubilizer, 0.05-5 parts by weight of the release-regulating agent, 20-40 parts by weight of the solvent, and 0-1 part by weight of the antioxidant; when the drug is selected from a nonsteroidal anti-inflammatory drug, the sustained-release analgesic pharmaceutical composition includes 1-5 parts by weight of the drug, 55-68 parts by weight of the delivery carrier, 0-3 parts by weight of the solubilizer, 0.05-5 parts by weight of the release-regulating agent, 20-40 parts by weight of the solvent, and 0-1 part by weight of the antioxidant.

Preferably, when the drug is selected from a local anesthetic and a nonsteroidal anti-inflammatory drug, the sustained-release analgesic pharmaceutical composition includes 0.01-15 parts by weight of the local anesthetic, 0.01-5 parts by weight of the nonsteroidal anti-inflammatory drug, 30-80 parts by weight of the delivery carrier, more than 0 and less than or equal to 10 parts by weight of the solubilizer, 0.05-20 parts by weight of the release-regulating agent, more than 0 and less than or equal to 70 parts by weight of the solvent, and 0-5 parts by weight of the antioxidant.

More preferably, when the drug is selected from a local anesthetic and a nonsteroidal anti-inflammatory drug, the sustained-release analgesic pharmaceutical composition includes 1-5 parts by weight of the local anesthetic, 0.01-3 parts by weight of the nonsteroidal anti-inflammatory drug, 50-70 parts by weight of the delivery carrier, more than 0 and less than or equal to 5 parts by weight of the solubilizer, 0.05-10 parts by weight of the release-regulating agent, 20-60 parts by weight of the solvent, and 0-2 parts by weight of the antioxidant.

Further preferably, when the drug is selected from a local anesthetic and a nonsteroidal anti-inflammatory drug, the sustained-release analgesic pharmaceutical composition includes 2-4 parts by weight of the local anesthetic, 0.01-2 parts by weight of the nonsteroidal anti-inflammatory drug, 55-68 parts by weight of the delivery carrier, more than 0 and less than or equal to 3 parts by weight of the solubilizer, 0.05-5 parts by weight of the release-regulating agent, 20-40 parts by weight of the solvent, and 0-1 part by weight of the antioxidant.

Preferably, the solvent is at least one selected from the group consisting of alcohols, N-methylpyrrolidone, benzyl benzoate, dimethyl sulfoxide, and glyceryl triacetate (triacetin).

Preferably, the alcohols include at least one of methanol, ethanol, n-propanol, isopropanol, n-butanol, isobutanol, tert-butanol, ethylene glycol, propylene glycol, glycerol, benzyl alcohol, phenethyl alcohol, and polyethylene glycol.

Preferably, the antioxidant is at least one selected from the group consisting of vitamin C (ascorbic acid), cysteine hydrochloride, vitamin E (tocopherol), ascorbate palmitate, glutathione, α-lipoic acid, thioglycerol, butylated hydroxytoluene, and butylated hydroxyanisole.

In a second aspect, the present disclosure provides a method for preparing the sustained-release analgesic pharmaceutical composition described in the first aspect of the present disclosure.

Specifically, the method for preparing the sustained-release analgesic pharmaceutical composition includes the step of:
mixing raw material components to obtain the sustained-release analgesic pharmaceutical composition.

Preferably, the method for preparing the sustained-release analgesic pharmaceutical composition is as follows:
mixing a local anesthetic and/or a nonsteroidal anti-inflammatory drug, a release-regulating agent, a solubilizer, and a solvent at a certain temperature, then adding a delivery carrier and an antioxidant and mixing to obtain the sustained-release analgesic pharmaceutical composition.

Preferably, the temperature is 50°C to 70°C; more preferably, the temperature is 55°C to 65°C.

Preferably, the method for preparing the sustained-release analgesic pharmaceutical composition is further as follows: mixing a release-regulating agent and a solvent, adding a delivery carrier and an antioxidant at a certain temperature to obtain a mixed solution, finally adding the local anesthetic and/or the non-steroidal anti-inflammatory drug and mixing to obtain the sustained-release analgesic pharmaceutical composition.

Preferably, the temperature is 40°C to 60°C; more preferably, the temperature is 45°C to 55°C.

Preferably, the mixed solution is heated before adding the anesthetic and the nonsteroidal anti-inflammatory drug.

Preferably, the temperature after heating is 50°C to 70°C; more preferably, the temperature after heating is 55°C to 65°C.

In a third aspect, the present disclosure provides a pharmaceutical preparation.

Specifically, the pharmaceutical preparation includes the sustained-release analgesic pharmaceutical composition described in the first aspect of the present disclosure.

Compared with the existing technologies, the beneficial effects of the technical solution provided in the present disclosure are as follows:
(1) The present disclosure uses a specific type of release-regulating agents (phospholipid compounds), which, together with the delivery carrier, can increase the in vivo plasma concentration upon the initial release, enhance the early analgesic efficacy and maintain sustained release. After administration to animals, when the drug contains a local anesthetic, the time to peak plasma concentration of the local anesthetic is 0.1 h to 8 h; when the drug is selected from a nonsteroidal anti-inflammatory drug, the time to peak plasma concentration is 1 h to 28 h, and even when the drug contains a local anesthetic, the time to peak plasma concentration of the local anesthetic is 0.4 h to 4 h; when the drug is selected from a nonsteroidal anti-inflammatory drug, the time to peak plasma concentration is 2 h to 10 h, which is short; it is exactly opposite to the existing technologies that delays the sustained release duration of the active pharmaceutical ingredient and has a longer time to reach peak drug release. Meanwhile, when both local anesthetics and non-steroidal anti-inflammatory drugs are present, the pharmaceutical composition still exhibits good analgesic effect 72 hours after surgery; when the drug is selected from a non-steroidal anti-inflammatory drug, the pharmaceutical composition still maintains a good analgesic effect from 24 h to 72 h after surgery, indicating that the drug in the pharmaceutical composition of the present disclosure has a good sustained release effect. Furthermore, the addition of phospholipids can also promote good wound healing.
(2) The addition of a specific type of solubilizers to the pharmaceutical composition of the present disclosure provides good stability for the drug, prevents precipitation, and makes it suitable for storage at room temperature and low temperature. The pharmaceutical composition remains a transparent and homogeneous solution after storage at room temperature for 24 hours without any precipitation. Moreover, it can avoid the adverse effects of organic acids or inorganic acids on the wound, without reducing the pH value of the wound or causing adverse impacts on wound inflammation and healing.
(3) The pharmaceutical composition of the present disclosure can overcome the shortcomings of treatment methods such as opioids and analgesia pumps, effectively relieve postoperative pain, reduce the frequency of administration, and simplify analgesic treatment methods.
(4) The pharmaceutical composition of the present disclosure can be administered directly through the postoperative wound, which is convenient to use. It forms a drug reservoir in the wound site to slowly release the drug, reducing the discomfort caused by the use of analgesia pumps or intravenous indwelling needles and improving patient compliance.

### BRIEF DESCRIPTION OF DRAWINGS

Fig. 1 shows the precipitation status of the pharmaceutical compositions of Example 14 and Comparative Examples 1-3 of the present disclosure;
Fig. 2 shows the plasma concentration curves of bupivacaine in SD rats after administration of the pharmaceutical compositions of Examples 13-15 and Comparative Example 4 of the present disclosure;
Fig. 3 shows the plasma concentration curves of meloxicam in SD rats after administration of the pharmaceutical compositions of Examples 13-15 and Comparative Example 4 of the present disclosure;
Fig. 4 is a schematic diagram of the incision site in the postoperative pain test of Bama pigs;
Fig. 5 shows the results of the Von Frey filament stimulation test in Bama pigs after administration of the pharmaceutical compositions of Example 14, Example 61, Comparative Example 4 and Comparative Example 5 of the present disclosure;
Fig. 6 shows the results of the Von Frey filament stimulation test in SD rats after administration of the pharmaceutical compositions of Example 14 and Comparative Example 4 of the present disclosure;
Fig. 7 shows the results of the plantar hot plate pain test of SD rats after administration of the pharmaceutical compositions of Example 68, Comparative Example 7 and Comparative Example 8 of the present disclosure;
Fig. 8 shows the histological section of wound tissues of Bama pigs after administration of the pharmaceutical composition of Example 14 of the present disclosure;
Fig. 9 shows the histological section of wound tissues of Bama pigs after administration of Zynrelef (the pharmaceutical composition of Comparative Example 5 of the present disclosure); and
Fig. 10 shows the histological section of wound tissues of Bama pigs after administration of the pharmaceutical composition of Comparative Example 4 of the present disclosure.

### DETAILED DESCRIPTION

To enable those skilled in the art to understand the technical solutions described in the present disclosure more clearly, the following examples are provided for illustration. It should be noted that the following examples do not constitute a limitation on the scope of protection claimed in the present disclosure.

Unless otherwise specified, the raw materials, reagents, or equipment used in the following examples are available from conventional commercial sources or can be obtained by existing known methods.

### Examples 1-33

The sustained-release analgesic pharmaceutical compositions of Examples 1-33, with their raw Material Components and Dosages (Parts by Weight), are shown in Table 1.

**Table 1: Raw Material Components and Dosages (Parts by Weight) of the Sustained-Release Analgesic Pharmaceutical Compositions of Examples 1-33**

| Example | Local anest hetic | Nons teroi dal anti-i nfla mmat ory drug | Solvent | | | Del iver y carr ier | Release-regulatin g agent | | | Solubilizer | | | Antiox idant |
|---|---|---|---|---|---|---|---|---|---|---|---|---|---|
| | bupiv acain e | Melo xica m | Dime thyl sulfo xide | Benz yl alcoh ol | Triac etin | Suc rose acet ate isob utyr ate | SP C | DS PC | EP C | Me nth ol | Bor neo l | Ca mp hor | Vitami n E |
| Example 1 | 1.29 | 0.08 | 33.63 | - | - | 64 | 0.5 | - | - | 0.5 | - | - | - |
| Example 2 | 2.57 | 0.08 | 32.35 | - | - | 64 | 0.5 | - | - | 0.5 | - | - | - |
| Example 3 | 3.86 | 0.08 | 31.06 | - | - | 64 | - | - | 0.5 | 0.5 | - | - | - |
| Example 4 | 1.29 | 0.08 | - | 33.61 | - | 64 | - | 0.5 | - | - | 0.5 | - | 0.02 |
| Example 5 | 2.57 | 0.08 | - | 31.85 | - | 64 | 1 | - | - | - | 0.5 | - | 0.02 |
| Example 6 | 3.86 | 0.08 | - | 30.53 | - | 64 | - | 1 | - | - | 0.5 | - | 0.02 |
| Example 7 | 2.57 | 0.04 | 31.87 | - | - | 64 | - | - | 1 | - | - | 0.5 | 0.02 |
| Example 8 | 2.57 | 0.08 | 32.33 | - | - | 64 | 0.5 | - | - | - | - | 0.5 | 0.02 |
| Example 9 | 2.57 | 0.16 | 32.25 | - | - | 64 | - | 0.5 | - | - | - | 0.5 | 0.02 |
| Example 10 | 2.57 | 0.04 | 10 | - | 22.59 | 64 | - | - | 0.5 | - | 0.2 | - | 0.1 |
| Example 11 | 2.57 | 0.08 | 10 | - | 22.65 | 64 | 0.5 | - | - | 0.1 | - | - | 0.1 |
| Example 12 | 2.57 | 0.16 | 10 | - | 22.47 | 64 | 0.5 | - | - | 0.2 | - | - | 0.1 |
| Example 13 | 1.29 | 0.08 | 7.5 | - | 26.91 | 64 | 0.1 | - | - | 0.1 | - | - | 0.02 |
| Example 14 | 2.57 | 0.08 | 7.5 | - | 25.67 | 64 | 0.1 | - | - | 0.1 | - | - | 0.02 |
| Example 15 | 3.86 | 0.08 | 7.5 | - | 24.26 | 64 | 0.1 | - | - | 0.1 | - | - | 0.02 |
| Example 16 | 1.29 | 0.08 | 7.5 | - | 40.33 | 50 | - | 0.5 | - | 0.2 | - | - | 0.1 |
| Example 17 | 2.57 | 0.08 | 7.5 | - | 38.95 | 50 | - | - | 0.5 | 0.3 | - | - | 0.1 |
| Example 18 | 3.86 | 0.08 | 7.5 | - | 7.96 | 80 | 0.5 | - | - | 0.5 | - | - | 0.1 |
| Example 19 | 1.29 | 0.32 | - | 7.5 | 39.74 | 50 | - | - | 1 | - | 0.1 | - | 0.05 |
| Example 20 | 2.57 | 0.32 | - | 7.5 | 38.46 | 50 | - | - | 1 | 0.1 | - | - | 0.05 |
| Example 21 | 3.86 | 0.32 | - | 7.5 | 7.17 | 80 | - | - | 1 | 0.1 | - | - | 0.05 |
| Example 22 | 1.29 | 0.04 | 5 | - | 37.52 | 55 | 1 | - | - | - | 0.1 | - | 0.05 |
| Example 23 | 2.57 | 0.04 | 5 | - | 36.24 | 55 | - | 1 | - | - | 0.1 | - | 0.05 |
| Example 24 | 3.86 | 0.04 | 5 | - | 34.95 | 55 | - | - | 1 | - | 0.1 | - | 0.05 |
| Example 25 | 1.29 | 0.08 | 5 | - | 22.88 | 70 | 0.5 | - | - | - | - | 0.2 | 0.05 |
| Example 26 | 2.57 | 0.08 | 5 | - | 21.6 | 70 | - | 0.5 | - | - | - | 0.2 | 0.05 |
| Example 27 | 3.86 | 0.08 | 5 | - | 20.31 | 70 | - | - | 0.5 | - | - | 0.2 | 0.05 |
| Example 28 | 1.29 | 0.08 | 33.83 | - | - | 64 | 0.2 | - | - | - | 0.5 | - | 0.05 |
| Example 29 | 2.57 | 0.08 | 32.55 | - | - | 64 | - | 0.2 | - | - | 0.5 | - | 0.05 |
| Example 30 | 3.86 | 0.08 | 31.26 | - | - | 64 | - | - | 0.2 | - | 0.5 | - | 0.05 |
| Example 31 | 1.29 | 0.08 | 32.98 | - | - | 64 | 0.6 | - | - | - | 1 | - | 0.05 |
| Example 32 | 2.57 | 0.08 | 31.7 | - | - | 64 | - | 0.6 | - | - | 1 | - | 0.05 |
| Example 33 | 3.86 | 0.08 | 30.41 | - | - | 64 | - | - | 0.6 | - | 1 | - | 0.05 |

The method for preparing the sustained-release analgesic pharmaceutical compositions of Examples 1-33 included the following steps:
At a temperature of 60°C, a local anesthetic, a nonsteroidal anti-inflammatory drug, a release-regulating agent, and a solubilizer were added to a solvent and stirred to dissolve; then the delivery carrier sucrose acetate isobutyrate and an antioxidant were added sequentially, and the mixture was stirred until a transparent and homogeneous solution was formed, and thus the sustained-release analgesic pharmaceutical composition was obtained.

### Examples 34-43

The sustained-release analgesic pharmaceutical compositions of Examples 34-43, with their raw Material Components and Dosages (Parts by Weight), are shown in Table 2.

**Table 2: Raw Material Components and Dosages (Parts by Weight) of the Sustained-Release Analgesic Pharmaceutical Compositions of Examples 34-43**

| Example | Local anest hetic | Nonste roidal anti-inf lammat ory drug | Solvent | | | Deliv ery carrie r | Release-regulatin g agent | | | Solubilizer | | | Anti oxid ant |
|---|---|---|---|---|---|---|---|---|---|---|---|---|---|
| | bupiv acain e | Celeco xib | Dime thyl sulfo xide | Benz yl alcoh ol | Triac etin | Sucro se acetat e isobu tyrate | SP C | DS PC | EP C | Me nth ol | Bor neo l | Ca mp hor | Vita min E |
| Example 34 | 1.29 | 0.09 | 17.5 | - | 16.82 | 64 | 0.1 | - | - | - | - | 0.2 | - |
| Example 35 | 2.57 | 0.09 | 17.5 | - | 15.54 | 64 | 0.1 | - | - | - | 0.2 | - | - |
| Example 36 | 2.57 | 0.09 | 17.5 | - | 15.24 | 64 | 0.5 | - | - | 0.1 | - | - | - |
| Example 37 | 3.86 | 0.09 | 7.5 | - | 23.85 | 64 | 0.5 | - | - | 0.2 | - | - | - |
| Example 38 | 1.29 | 0.09 | 7.5 | - | 26.52 | 64 | 0.5 | - | - | 0.1 | - | - | - |
| Example 39 | 2.57 | 0.05 | 7.5 | - | 25.28 | 64 | 0.5 | - | - | 0.1 | - | - | - |
| Example 40 | 2.57 | 0.18 | 32.63 | - | - | 64 | 0.5 | - | - | 0.1 | - | - | 0.02 |
| Example 41 | 1.29 | 0.09 | - | 17.5 | 16.4 | 64 | - | 0.5 | - | - | - | 0.2 | 0.02 |
| Example 42 | 2.57 | 0.09 | - | 20 | 12.62 | 64 | - | - | 0.5 | - | 0.2 | - | 0.02 |
| Example 43 | 1.29 | 0.09 | - | 15 | 18.9 | 64 | 0.5 | - | - | 0.2 | - | - | 0.02 |

The method for preparing the sustained-release analgesic pharmaceutical compositions of Examples 34-43 was the same as that of Examples 1-33.

### Examples 44-53

The sustained-release analgesic pharmaceutical compositions of Examples 44-53, with their raw material components and dosages (parts by weight), are shown in Table 3.

**Table 3: Raw Material Components and Dosages (Parts by Weight) of the Sustained-Release Analgesic Pharmaceutical Compositions of Examples 44-53**

| Example | Local anesth etic | Nonste roidal anti-inf lammat ory drug | Solvent | | | Deliver y carrier | Release-regulati ng agent | | | Solubilizer | | |
|---|---|---|---|---|---|---|---|---|---|---|---|---|
| | Ropiv acaine | Meloxi cam | Dimet hyl sulfox ide | Benz yl alco hol | Triace tin | Sucrose acetate isobuty rate | SP C | DS PC | EP C | Ment hol | Born eol | Cam phor |
| Example 44 | 2.57 | 0.09 | 33.14 | - | - | 64 | 0.1 | - | - | 0.1 | - | - |
| Example 45 | 2.57 | 0.09 | 33.04 | - | - | 64 | 0.1 | - | - | - | 0.2 | - |
| Example 46 | 2.57 | 0.09 | 32.74 | - | - | 64 | 0.5 | - | - | - | - | 0.1 |
| Example 47 | 2.57 | 0.09 | 17.5 | - | 15.14 | 64 | - | 0.5 | - | 0.2 | - | - |
| Example 48 | 1.29 | 0.09 | 17.5 | - | 16.52 | 64 | - | - | 0.5 | | 0.1 | - |
| Example 49 | 2.57 | 0.05 | 17.5 | - | 15.28 | 64 | 0.5 | - | - | 0.1 | - | - |
| Example 50 | 2.57 | 0.18 | 7.5 | - | 25.15 | 64 | 0.5 | - | - | 0.1 | - | - |
| Example 51 | 2.57 | 0.09 | - | 7.5 | 39.14 | 50 | - | 0.5 | - | - | - | 0.2 |
| Example 52 | 2.57 | 0.09 | - | 10 | 36.64 | 50 | - | - | 0.5 | - | 0.2 | |
| Example 53 | 3.86 | 0.09 | 7.5 | - | 38.45 | 50 | - | - | - | - | - | 0.1 |

The method for preparing the sustained-release analgesic pharmaceutical compositions of Examples 44-53 included the following steps:
A release-regulating agent was added to a solvent (dimethyl sulfoxide or benzyl alcohol), stirred to dissolve at a temperature of 50°C; then the delivery carrier sucrose acetate isobutyrate, triacetin, and an antioxidant were added while stirring until a transparent and homogeneous solution was formed; then the mixed solution was heated to 60°C, the non-steroidal anti-inflammatory drug meloxicam and local anesthetic ropivacaine were added to the solution, stirred until a transparent and homogeneous solution was formed, thus the sustained-release analgesic pharmaceutical composition was obtained.

### Examples 54-62

The sustained-release analgesic pharmaceutical compositions of Examples 54-62, with their raw material components and dosages (parts by weight), are shown in Table 4.

**Table 4: Raw Material Components and Dosages (Parts by Weight) of the Sustained-Release Analgesic Pharmaceutical Compositions of Examples 54-62**

| Example | Local anest hetic | Solvent | | | Deliver y carrier | Release-regulatin g agent | | | Solubilizer | | | Antiox idant |
|---|---|---|---|---|---|---|---|---|---|---|---|---|
| | bupiv acain e | Dimeth yl sulfoxi de | Benz yl alcoh ol | Triacet in | Sucros e acetate isobuty rate | SP C | DS PC | EP C | Me nth ol | Born eol | Ca mp hor | Vitami n E |
| Example 54 | 1.29 | 33.71 | - | - | 64 | 0.5 | - | - | 0.5 | - | - | - |
| Example 55 | 1.29 | - | 33.71 | - | 64 | 0.5 | - | - | 0.5 | - | - | - |
| Example 56 | 2.57 | 32.43 | - | - | 64 | - | - | 0.5 | - | 0.5 | - | - |
| Example 57 | 1.29 | 10 | - | 37.69 | 50 | - | 0.5 | - | - | 0.5 | - | 0.02 |
| Example 58 | 2.57 | - | 7.5 | 38.41 | 50 | 1 | - | - | - | 0.5 | - | 0.02 |
| Example 59 | 2.57 | - | 10 | 36.81 | 50 | - | 0.5 | - | 0.1 | - | - | 0.02 |
| Example 60 | 2.57 | 7.5 | - | 25.31 | 64 | - | 0.5 | - | - | 0.1 | - | 0.02 |
| Example 61 | 2.57 | 7.5 | - | 25.31 | 64 | 0.1 | - | - | 0.1 | - | - | 0.02 |
| Example 62 | 3.86 | 7.5 | - | 23.42 | 64 | 1 | - | - | 0.2 | - | - | 0.02 |

The method for preparing the sustained-release analgesic pharmaceutical compositions of Examples 54-62 included the following steps:
At a temperature of 60°C, the local anesthetic bupivacaine, a release-regulating agent, and a solubilizer were added to a solvent, stirred to dissolve, then the delivery carrier sucrose acetate isobutyrate and an antioxidant were added sequentially, and stirred until a transparent and homogeneous solution was formed, thus the sustained-release analgesic pharmaceutical composition was obtained.

### Examples 63-70

The sustained-release analgesic pharmaceutical compositions of Examples 63-70, with their raw material components and dosages (parts by weight), are shown in Table 5.

**Table 5: Raw Material Components and Dosages (Parts by Weight) of the Sustained-Release Analgesic Pharmaceutical Compositions of Examples 63-70**

| Example | Nonsteroidal anti-inflamma tory drug | Solvent | | | Delivery carrier | Release-regulating agent | | |
|---|---|---|---|---|---|---|---|---|
| | Meloxicam | Dimethyl sulfoxide | Benzyl alcohol | Triacetin | Sucrose acetate isobutyrate | SPC | DSP C | EPC |
| Example 63 | 1.34 | 47.46 | - | - | 50 | 0.2 | - | - |
| Example 64 | 2.68 | 45.97 | - | - | 50 | 0.35 | - | - |
| Example 65 | 2.68 | 26.94 | - | 19.18 | 50 | - | 0.2 | - |
| Example 66 | 1.34 | 38.18 | - | 20.24 | 39.74 | - | 0.5 | - |
| Example 67 | 2.68 | 38.2 | - | 18.88 | 39.74 | - | - | 0.5 |
| Example 68 | 2.68 | 38.18 | - | 18.4 | 39.74 | 0.5 | - | - |
| Example 69 | 2.68 | - | 47.94 | 18.88 | 30 | 0.5 | - | - |
| Example 70 | 1.34 | - | 47.92 | 19.74 | 30 | - | 1 | - |

The method for preparing the sustained-release analgesic pharmaceutical compositions of Examples 63-70 included the following steps:
At a temperature of 60°C, the non-steroidal anti-inflammatory drug meloxicam and a release-regulating agent were added to a solvent and stirred to dissolve, then the delivery carrier sucrose acetate isobutyrate and an antioxidant were added sequentially, and stirred until a transparent and homogeneous solution was formed, thus the sustained-release analgesic pharmaceutical composition was obtained.

### Comparative Example 1

The only difference between Comparative Example 1 and Example 14 was that the solubilizer menthol was not added in Comparative Example 1, and the dosage of the solvent triacetic acid was 25.77 parts; other conditions were the same as Example 14.

### Comparative Example 2

The only difference between Comparative Example 2 and Example 14 was that, in Comparative Example 2, an equal amount of the solubilizer arginine was used to replace menthol in Example 14; other conditions were the same as Example 14.

### Comparative Example 3

The only difference between Comparative Example 3 and Example 14 was that, in Comparative Example 3, an equal amount of the solubilizer maleic acid was used to replace menthol in Example 14; other conditions were the same as Example 14.

### Comparative Example 4

The only difference between Comparative Example 4 and Example 14 was that, in Comparative Example 4, an equal amount of the release-regulating agent castor oil was used to replace the SPC in Example 14; other conditions were the same as Example 14.

### Comparative Example 5

Comparative Example 5 used Zynrelef, a drug marketed overseas.

### Comparative Example 6

The only difference between Comparative Example 6 and Example 68 was that, in Comparative Example 6, an equal amount of the release-regulating agent sesame oil was used to replace the SPC in Example 68; other conditions were the same as Example 68.

### Comparative Example 7

Comparative Example 7 used Meloxicam Injection (Qilu Pharmaceutical Co., Ltd.), a drug marketed in China.

### Comparative Example 8

Comparative Example 8 used Meloxicam Tablets (Shanghai Boehringer Ingelheim Pharmaceuticals Co., Ltd.), a drug marketed in China.

### Performance Tests

### 1. Observation of drug precipitation

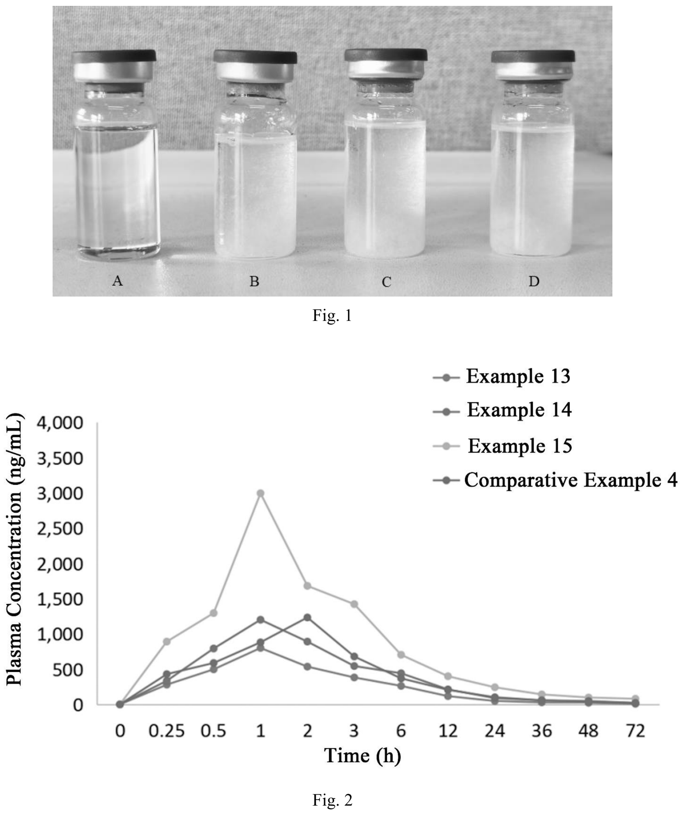

The pharmaceutical compositions of Example 14 and Comparative Examples 1-3 were stored at room temperature (25°C) for 24 hours, and the precipitation status was observed. The results are shown in Fig. 1, where A in Fig. 1 shows the precipitation status of Example 14, B, C, and D in Fig. 1 show the precipitation status of Comparative Examples 1-3, respectively.

As shown in from Fig. 1, the pharmaceutical composition of Example 14 with the addition of the solubilizer menthol remained a transparent and homogeneous solution without any precipitation after storage at room temperature (25°C) for 24 hours. In contrast, the pharmaceutical compositions of Comparative Example 1 (without solubilizer), Comparative Example 2 (added with arginine as a solubilizer), and Comparative Example 3 (added with maleic acid as a solubilizer) turned turbid after storage at room temperature (25°C) for 24 hours, with precipitation of the local anesthetic bupivacaine observed. The results indicated that the addition of menthol as a solubilizer to the pharmaceutical composition of the present disclosure can increase the solubility of the active pharmaceutical ingredient, preventing drug precipitation during the preparation process and sustained storage at room temperature, thus exhibiting good stability.

### 2. Test of time to peak plasma concentration

(1) To determine the time to peak plasma concentration (Tₘₐₓ) of the drug in the pharmaceutical compositions of the present disclosure, i.e., the time to onset of postoperative analgesia, the in vivo pharmacokinetic study of the pharmaceutical compositions of Examples 13-15 and Comparative Example 4 was conducted in rats. The specific process was as follows:
   Male SD rats weighing approximately 200 g to 250 g each were selected and subcutaneously administered with the pharmaceutical compositions of Examples 13-15 and Comparative Example 4 at the backs, with a dosage of 30.0 mg/kg. Approximately 0.5 mL of blood samples were collected from the SD rats at 0.25, 0.5, 1, 2, 3, 6, 12, 24, 48, 72, and 96 hours after administration, and placed in EDTA-2K+ anticoagulant blood collection tubes. The whole blood was centrifuged at 8,000 rpm for 5 min to collect plasma, and then the drug concentration in the plasma samples was detected by liquid chromatography-tandem mass spectrometry (LC-MS/MS).

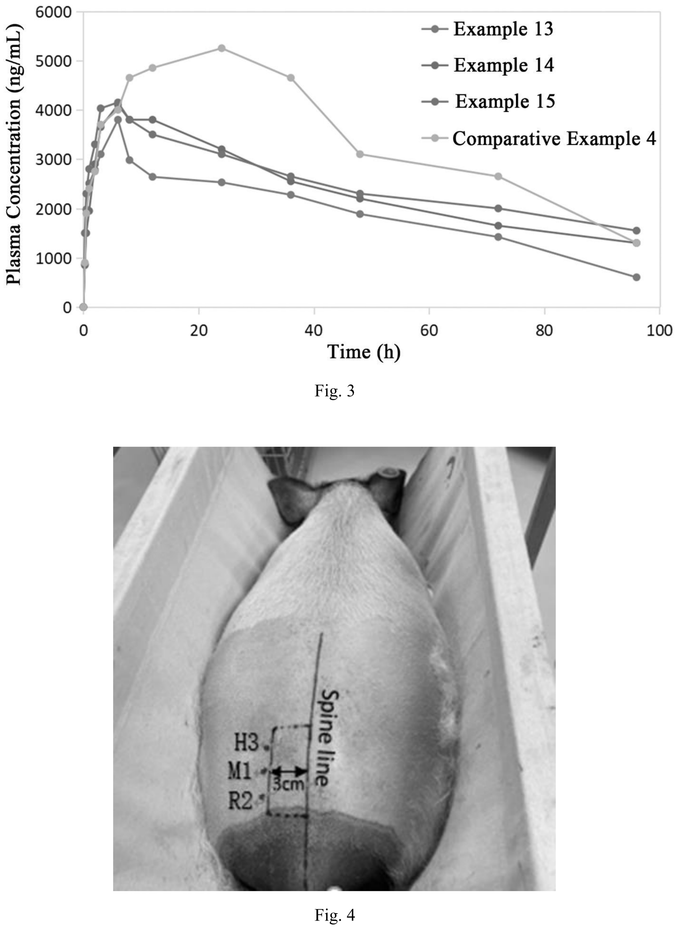

The plasma concentration curves of the local anesthetic bupivacaine and the nonsteroidal anti-inflammatory drug meloxicam after administration of the pharmaceutical compositions of Examples 13-15 and Comparative Example 4 are shown in Fig. 2 and Fig. 3, respectively.

As can be seen from Fig. 2 and Fig. 3, after the addition of the release-regulating agent (SPC) in the pharmaceutical compositions of Examples 13-15, the Tₘₐₓ of bupivacaine in SD rats was 1 hour, and the Tₘₐₓ of meloxicam was 6 hours. In Comparative Example 4, after the addition of castor oil as the release-regulating agent in the pharmaceutical composition, the Tₘₐₓ of bupivacaine in SD rats was 2 hours, and the Tₘₐₓ of meloxicam was 24 hours. The results indicated that, the addition of phospholipid compounds as the release-regulating agent in the pharmaceutical composition of the present disclosure, in combination with other components, could shorten the Tₘₐₓ of the drugs, accelerate the onset of postoperative analgesia. Furthermore, the pharmaceutical compositions of Examples 13-15 all exhibited sustained-release effects with stable and long-lasting release, which could significantly alleviate patients' pain and discomfort in the early postoperative period and reduce administration frequency. The technical effect of shortening the Tₘₐₓ and accelerating the onset of postoperative analgesia by adding phospholipid compounds as the release-regulating agent in the pharmaceutical composition is exactly opposite to the conventional theoretical knowledge and technical enlightenment in the existing technologies that release-regulating agents can delay the sustained release duration of the active pharmaceutical ingredient.

(2) Male SD rats weighing approximately 200 g to 250 g each were selected and subcutaneously administered with the pharmaceutical compositions of Example 68 and Comparative Example 6 at the backs, with a dosage of 30.0 mg/Kg. Approximately 0.5 mL of blood samples were collected from the SD rats at 0.5, 1, 2, 4, 8, 12, 24, 48, 72, and 96 hours after administration, and placed in EDTA-2K+ anticoagulant blood collection tubes. The whole blood was centrifuged at 8,000 rpm for 5 min to collect plasma, and then the drug concentration in the plasma samples was detected by LC-MS/MS. The plasma concentration data of meloxicam in SD rats after administration of the pharmaceutical compositions of Example 68 and Comparative Example 6 are shown in Table 6.

**Table 6: Plasma Concentration Data of Meloxicam in SD Rats after Administration of the Pharmaceutical Compositions of Example 68 and Comparative Example 6**

| Example/Comparative Example | Cₘₐₓ | Tₘₐₓ | T_{1/2} |
|---|---|---|---|
| Example 68 | 8934 | 4 | 32.5 |
| Comparative Example 6 | 8608 | 8 | 26.8 |

Cₘₐₓ represents the peak plasma concentration; Tₘₐₓ represents the time to peak plasma concentration; T_{1/2} represents the half-life of the drug.

As can be seen from Table 6, after the addition of the release-regulating agent (SPC) in the pharmaceutical composition of Example 68, the Tₘₐₓ of meloxicam in SD rats was 4 hours. In Comparative Example 6, the Tₘₐₓ of meloxicam in SD rats was 8 hours. The results indicated that the addition of phospholipid compounds as the release-regulating agent in the pharmaceutical composition of the present disclosure, in combination with other components, could shorten the Tₘₐₓ of the drug, accelerate the onset of postoperative analgesia, and the pharmaceutical composition of Example 68 exhibited sustained-release effect (as shown in Table 7 below, compared with Comparative Examples 7 and 8, the release was more prolonged in Example 68), which could significantly alleviate patients' pain and discomfort for a longer time after surgery and reduce administration frequency.

### 3. Analgesic Effect Test

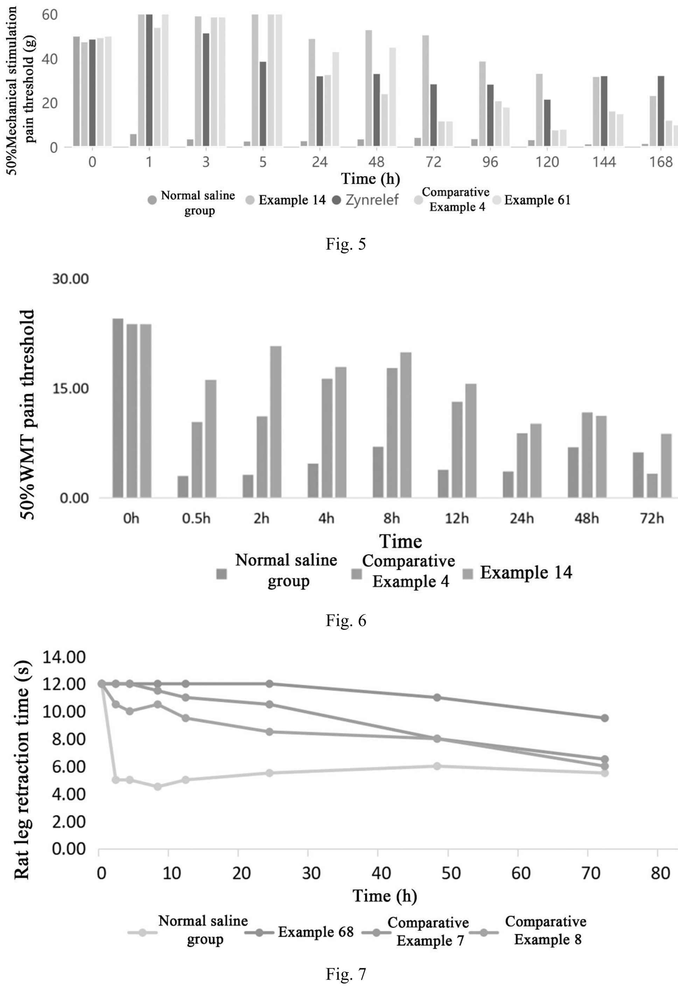

(1) The analgesic effects of the pharmaceutical compositions of Example 14, Example 61, Comparative Example 4, and Comparative Example 5 (Zynrelef) were tested using a Bama pig postoperative pain model. The specific process was as follows:
   Bama miniature pigs weighing 8 Kg to 12 Kg each were selected and anesthetized with isoflurane. An incision of 7 cm in length was made on the lower back of each pig, 3 cm to the left of the spine, deep to the fascial layer, with separation of the skin and muscle layers on both sides. Pigs in each group received direct infusion of the pharmaceutical composition at the wound site, and each Bama miniature pig was administered with normal saline, the pharmaceutical composition of Example 14, Example 61, Comparative Example 4, or Comparative Example 5 at a dose of 100 mg/kg, respectively, followed by wound suturing. The Von Frey filament stimulation test was performed on each group of Bama miniature pigs before administration (0 h) and at 1, 3, 5, 7, 12, 24, 48, 72, 96, 120, 144, and 168 hours after administration using a Von Frey anesthesiometer with 9 intensities of Von Frey filaments (equivalent to 1.4, 2, 4, 6, 8, 10, 15, 26, and 60 g, respectively). The "up-and-down" method was used to detect and record the test results and calculate the pain threshold. The schematic diagram of the incision position in the postoperative pain test of Bama miniature pigs is shown in Fig. 4, where H3, M1, and R2 represented the test points, and the spine line represented the midline of the back of the Bama miniature pig. The results of the Von Frey filament stimulation test within 168 hours after administration of the pharmaceutical compositions of Example 14, Example 61, Comparative Example 4, and Comparative Example 5 are shown in Fig. 5; the smaller the ordinate value, the more obvious the pain and the lower the drug efficacy.

As can be seen from Fig. 5, at 24 hours after surgery, the analgesic effects of Zynrelef in Comparative Example 5 and the pharmaceutical composition in Comparative Example 4 began to weaken; moreover, from 1 to 3 days after surgery, the analgesic effects of Comparative Examples 5 and 4 were significantly worse than that of the pharmaceutical composition of Example 14 in the present disclosure. The results indicated that the pharmaceutical composition of Example 14 in the present disclosure had a better and longer-lasting postoperative analgesic effect than the marketed drug Zynrelef.

The analgesic effect of the pharmaceutical composition of Example 14 was basically the same as that of Example 61 within 48 hours after surgery; from 72 hours onwards, the drug efficacy of Example 61 began to decline, while that of Example 14 remained effective, indicating that the addition of meloxicam to the formulation of the present disclosure could achieve a longer-lasting postoperative analgesic effect.

(2) The analgesic effect of the pharmaceutical compositions of Example 14 and Comparative Example 4 was tested using a postoperative pain model of SD rats. The specific process was as follows:
Specific pathogen-free (SPF) SD rats (5-6 weeks old, male) were selected and anesthetized with isoflurane. An incision of 1.0 cm in length was made on the plantar surface, cutting through the muscle without affecting its origin, insertion, and attachment. After suturing, the pharmaceutical composition was administered by infiltration injection beside the incision, and each SD rat was administered with normal saline, the pharmaceutical composition of Example 14, or Comparative Example 4 at a dose of 30 mg/Kg. The Von Frey mechanical hyperalgesia test was performed on each SD rat before surgical administration (0 h) and at 0.5, 2, 4, 8, 12, 24, 48, and 72 hours after administration using Von Frey filaments with 9 intensities (equivalent to 1, 1.4, 2, 4, 6, 8, 10, 15, and 26 g, respectively). The "up-and-down" method was used to detect and record the test results and calculate the pain threshold. The results of the Von Frey filament stimulation test within 72 hours after administration of the pharmaceutical compositions of Example 14 and Comparative Example 4 are shown in Fig. 6; the smaller the ordinate value, the more obvious the pain and the lower the drug efficacy.

As can be seen from Fig. 6, at 0.5 h and 2.0 h after surgery, the analgesic effect of the pharmaceutical composition of Example 14 was significantly better than that of Comparative Example 4; as time progressed, the analgesic effect of Example 14 was also slightly better than that of Comparative Example 4 from 4 h to 48 h after surgery. The results indicated that the pharmaceutical composition of Example 14 in the present disclosure had a better postoperative analgesic effect than Comparative Example 4, especially in the early postoperative period; moreover, as time progressed, the overall analgesic effect of the pharmaceutical composition of Example 14 was also superior to that of Comparative Example 4. It could be seen that the analgesic effect of the pharmaceutical composition added with phospholipid compounds as the release-regulating agent was significantly better than that added with other release-regulating agents (e.g., castor oil), especially in the early postoperative period.

(3) The analgesic effects of the pharmaceutical compositions of Example 68, Comparative Example 7 and Comparative Example 8 were tested using a postoperative pain model of SD rats. The specific process was as follows:
Specific pathogen-free (SPF) SD rats (5-6 weeks old, male) were selected and anesthetized with isoflurane. An incision of 1.0 cm in length was made on the plantar surface and sutured, followed by infiltration injection of the pharmaceutical composition beside the incision. Each SD rat was administered with normal saline, the pharmaceutical composition of Example 68, Comparative Example 7, or Comparative Example 8 at a dose of 30 mg/Kg. The plantar hot plate pain test was performed before surgical administration (0 h) and at 0.5, 2, 4, 8, 12, 24, 48, and 72 hours after administration. The hot plate instrument was turned on (temperature set at 56°C), and after the temperature of the hot plate stabilized, the surgically treated foot of the rat was placed on the hot plate. Timing started when the rat's foot touched the hot plate and ended when the rat lifted its foot for the first time, and the tolerance time of the rat on the hot plate was recorded (in seconds). Each rat was tested 3 times with an interval of not less than 30 seconds between each test. The cut-off time was set at 12 seconds; rats that did not lift their feet after 12 seconds were considered insensitive to thermal pain and no longer tested. The average value of the 3 measurements was taken as the test result at a specific time point.

The results of the plantar hot plate pain test in SD rats within 72 hours after administration of the pharmaceutical compositions of Example 68, Comparative Example 7, and Comparative Example 8 are shown in Fig. 7; The smaller the ordinate value, the more obvious the pain and the lower the drug efficacy. Meanwhile, at the same time points, i.e., before surgical administration (0 h) and at 0.5, 2, 4, 8, 12, 24, 48, and 72 hours after administration, approximately 0.5 mL of blood samples were collected from the SD rats and placed in EDTA-2K+ anticoagulant blood collection tubes. The whole blood was centrifuged at 8,000 rpm for 5 min to collect plasma, and then the drug concentration in the plasma samples was detected by LC-MS/MS. The plasma concentration data in SD rats after administration of the pharmaceutical compositions of Example 68, Comparative Example 7, and Comparative Example 8 are shown in Table 7.

As shown in Fig. 7, within 24 hours after surgery, the analgesic effect of the pharmaceutical composition of Example 68 was better than that of Comparative Example 7; as time progressed, from 24 h to 72 h after surgery, the analgesic effect of the pharmaceutical composition of Example 68 was significantly better than that of Comparative Example 7, still maintaining a high pain threshold, indicating that compared with Comparative Example 7, the pharmaceutical composition in Example 68 had a longer-lasting postoperative analgesic effect in SD rats. It could be seen that the pharmaceutical composition of the present disclosure was superior to the marketed immediate-release dosage form (injection) of anti-inflammatory drugs, especially in terms of the analgesic effect from 24 h to 72 h after surgery.

In addition, the analgesic effect of the pharmaceutical composition of Example 68 was also significantly better than that of Comparative Example 8, with a significantly longer-lasting analgesic effect, indicating that the injection administration method of the pharmaceutical composition of the present disclosure is superior to the marketed immediate-release oral dosage form.

**Table 7: Plasma Concentration Data in SD Rats after Administration of the Pharmaceutical Compositions of Example 68, Comparative Example 7, and Comparative Example 8**

| Example/Comparative Example | Drug | Cₘₐₓ | T_{1/2} |
|---|---|---|---|
| Example 68 | Meloxicam | 8312 | 31.0 |
| Comparative Example 7 | Meloxicam Injection | 10354 | 8.9 |
| Comparative Example 8 | Meloxicam Tablets (oral) | 4191 | 11.7 |

Cₘₐₓ represents the peak plasma concentration; T_{1/2} represents the half-life of the drug.

As shown in Table 7, compared with the marketed intermediate-release injection or oral dosage forms, the pharmaceutical composition of the present disclosure had a higher peak plasma concentration and a longer half-life, indicating that the pharmaceutical composition of Example 68 in the present disclosure has a good analgesic effect and a longer-lasting effect in the early postoperative period.

### 4. Skin Healing Test

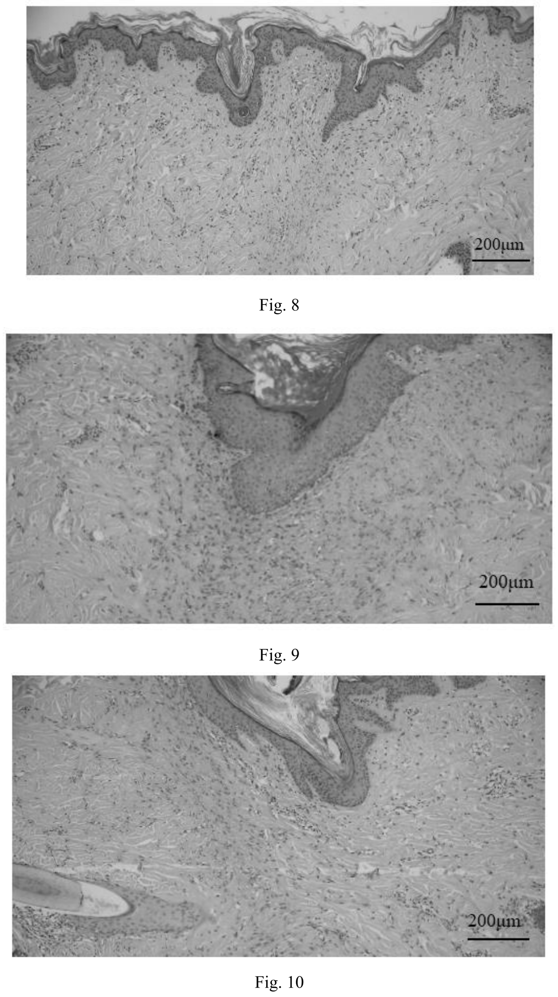

A skin healing test was conducted to evaluate the skin healing effects of the pharmaceutical compositions of Example 14, Comparative Example 5 (Zynrelef), and Comparative Example 4 (with castor oil as the release-regulating agent). The specific process was as follows:
In the analgesic effect test, on the 16th day after administration, wound tissue sections were collected from Bama pigs administered with the pharmaceutical compositions of Example 14, Comparative Example 4, and Comparative Example 5, stained with hematoxylin-eosin (HE staining) and observed. The wound tissue sections of Bama pigs after administration of the pharmaceutical compositions of Example 14, Comparative Example 5, and Comparative Example 4 are shown in Fig. 8, Fig. 9, and Fig. 10, respectively.

As shown in Fig. 8, 16 days after administration of the pharmaceutical composition of Example 14, two vertical deep wounds were observed from the epidermis to the subcutaneous adipose tissue, both of which were closed. The wound surface was covered by the epidermis; the epidermal cell layer increased to 10-12 layers at one site and 5-7 layers at the other site, with a small amount of crust remaining on the epidermal surface. Vertical linear fibrous tissue hyperplasia was observed in the dermal wound, with a large number of fibroblasts, moderate collagen fiber formation, a small amount of mucoid matrix deposition, insignificant capillary hyperplasia (1-3 vascular cross-sections per high-power field (HPF)), a small amount of red blood cell extravasation, and insignificant infiltration of lymphocytes and histiocytes (1-4 per HPF); no obvious infiltration of neutrophils and eosinophils was observed. Fibrous hyperplasia was present in the subcutaneous adipose tissue, with a small amount of adipose tissue necrosis and dissolution, forming granulomatous structures and focal dense lymphocyte infiltration; no foreign bodies were observed in the wound.

As shown in Fig. 9, 16 days after administration of the pharmaceutical composition of Comparative Example 5, two vertical deep wounds were observed from the epidermis to the subcutaneous adipose tissue, both of which were closed; the wound surface was covered by the epidermis, with the epidermal cell layer increased to 10-12 layers and a small amount of crust remaining on the epidermis. Vertical linear fibrous tissue hyperplasia was observed in the dermal wound, with a large number of fibroblasts, moderate collagen fiber formation, a small amount of mucoid matrix deposition, mild capillary hyperplasia (5-8 vascular cross-sections per HPF), a small amount of red blood cell extravasation, and insignificant infiltration of lymphocytes and histiocytes (2-5 per HPF). No obvious infiltration of neutrophils and eosinophils was observed. Fibrous hyperplasia was present in the subcutaneous adipose tissue, with a small amount of adipose tissue necrosis and dissolution, forming granulomatous structures, and insignificant lymphocyte infiltration; no foreign bodies were observed in the wound.

As shown in Fig. 10, 16 days after administration of the pharmaceutical composition of Comparative Example 4, one oblique wound located in the dermis extending from the epidermis to the subcutaneous adipose tissue was observed, which was closed. The wound surface was covered by the epidermis, with the epidermal cell layer increased to 8-10 layers and a small amount of scab remaining on the surface; neutrophils and necrotic debris were detected within the scab. Vertical linear fibrous tissue hyperplasia was observed in the dermal wound, with a number of fibroblasts, moderate collagen fiber formation, and the hyperplasia width was greater than that in Group C. A small amount of mucoid matrix deposition and mild capillary hyperplasia (5-8 vascular cross-sections per HPF) were observed, along with slight erythrocyte extravasation. Infiltration of lymphocytes and histiocytes was minimal (2-9 per HPF), and no neutrophils or eosinophils were detected. Hair follicles around the wound were deformed and distorted due to fibrous hyperplasia. In the subcutaneous adipose tissue, extensive fibrous hyperplasia was noted, accompanied by multifocal dense lymphocyte infiltration and scattered mild lymphocyte infiltration (11-15 per HPF), with occasional eosinophil infiltration and no neutrophil infiltration. No foreign bodies were observed in the wound.

The HE staining results of postoperative tissue sections from Bama pigs demonstrated that the pharmaceutical composition of Example 14 in this application exhibited excellent performance in promoting skin wound healing. In terms of skin healing efficacy, it outperformed the marketed drug Zynrelef (Comparative Example 5) and the pharmaceutical composition using non-phospholipid release-regulating agents (Comparative Example 4). Pathological observations also confirmed that it has superior performance in reducing vascular hyperplasia and alleviating inflammatory responses compared to Comparative Example 5 and Comparative Example 4. These results indicated that the use of phospholipid compounds as release-regulating agents could achieve a good effect in accelerating skin wound healing and repair.

In addition, the pharmaceutical compositions of other examples in this application also demonstrated the advantages of shortening the time to peak plasma concentration, accelerating the onset of postoperative analgesia, providing good analgesic effect in the early postoperative period, and enabling sustained release of the drug. Meanwhile, the pharmaceutical compositions had good stability and efficacy in promoting skin healing.

In summary, the use of phospholipid compounds as release-regulating agents in this application can increase the initial plasma concentration of local anesthetics and non-steroidal anti-inflammatory drugs in vivo, achieve enhanced analgesic effect in the early postoperative stage, relieve postoperative pain in patients, maintain sustained release, and promote skin wound healing and repair. Furthermore, the present disclosure uses a specific type of solubilizer, which provides good stability for the drug and prevents precipitation. The addition of phospholipid release-regulating agent to the pharmaceutical composition of the present disclosure can shorten the time to peak plasma concentration and accelerate the onset of postoperative analgesia. This technical effect is exactly opposite to the conventional theoretical knowledge and existing technologies teachings that release-regulating agents can delay the sustained release duration of the active pharmaceutical ingredient. This is an unexpected discovery made by the inventors through continuous experimental exploration.

The foregoing examples are merely used to illustrate the technical solutions of this application rather than limit the scope of protection of the present disclosure. Although the present disclosure has been described in detail with reference to preferred examples, those skilled in the art should understand that modifications or equivalent substitutions can be made to the technical solutions of the present disclosure without departing from the essence and scope of the technical solutions of the present disclosure.

## Claims

1. A pharmaceutical composition, **characterized by** comprising a drug, a delivery carrier, and a release-regulating agent;
wherein the drug is at least one selected from the group consisting of a local anesthetic and a nonsteroidal anti-inflammatory drug;
wherein the release-regulating agent is selected from a phospholipid compound; and
wherein the delivery carrier is selected from the group consisting of a saccharide and an esterified form thereof.

2. The pharmaceutical composition according to claim 1, **characterized in that** when the drug contains the local anesthetic, the pharmaceutical composition further contains a solubilizer; preferably, the solubilizer is at least one selected from the group consisting of menthol, camphor, borneol, linalool, and eucalyptol.

3. The pharmaceutical composition according to claim 1, **characterized in that** the local anesthetic is selected from an amide anesthetic; preferably, the amide anesthetic is at least one selected from the group consisting of bupivacaine, ropivacaine, and a pharmaceutically acceptable salt or a stereoisomer thereof; the nonsteroidal anti-inflammatory drug is at least one selected from the group consisting of meloxicam, celecoxib, and a pharmaceutically acceptable salt or a stereoisomer thereof.

4. The pharmaceutical composition according to claim 1, **characterized in that** the delivery carrier is at least one selected from the group consisting of sucrose, chitosan, sucrose acetate isobutyrate, sucrose octaacetate, and sucrose monoacetate monoisobutyrate.

5. The pharmaceutical composition according to claim 1, **characterized in that** the phospholipid compound is at least one selected from the group consisting of a natural phospholipid and a synthetic phospholipid; preferably, the natural phospholipid is at least one selected from the group consisting of a soybean phosphatidylcholine, an egg yolk phosphatidylcholine, a rapeseed phospholipid, and a sunflower phospholipid; and the synthetic phospholipid is at least one selected from the group consisting of dierucoyl phosphatidylcholine, dioleoyl phosphatidylcholine, palmitoyl oleoyl phosphatidylcholine, distearoyl phosphatidylcholine, dipalmitoyl phosphatidylglycerol, and distearoyl phosphatidylglycerol.

6. The pharmaceutical composition according to any one of claims 1 to 5, **characterized in that** after administration of the pharmaceutical composition to an animal, when the drug contains the local anesthetic, the time to peak plasma concentration of the local anesthetic is 0.1 h to 8 h; when the drug is selected from a nonsteroidal anti-inflammatory drug, the time to peak plasma concentration is 1 h to 28 h.

7. The pharmaceutical composition according to claim 1, **characterized in that** the pharmaceutical composition comprises 0.01-20 parts by weight of the drug, 30-80 parts by weight of the delivery carrier, and 0.05-20 parts by weight of the release-regulating agent.

8. The pharmaceutical composition according to claim 2, **characterized in that** when the drug contains the local anesthetic, the pharmaceutical composition comprises 0.01-20 parts by weight of the drug, 30-80 parts by weight of the delivery carrier, more than 0 and less than or equal to 10 parts by weight of the solubilizer, and 0.05-20 parts by weight of the release-regulating agent.

9. The pharmaceutical composition according to any one of claims 1 to 5, **characterized in that** the pharmaceutical composition further comprises a solvent; or, the pharmaceutical composition further comprises a solvent and an antioxidant.

10. The pharmaceutical composition according to claim 9, **characterized in that** the pharmaceutical composition comprises 0.01-20 parts by weight of the drug, 30-80 parts by weight of the delivery carrier, 0.05-20 parts by weight of the release-regulating agent, more than 0 and less than or equal to 70 parts by weight of the solvent, and 0-5 parts by weight of the antioxidant.

11. The pharmaceutical composition according to claim 10, **characterized in that** when the drug contains the local anesthetic, the pharmaceutical composition comprises 0.01-20 parts by weight of the drug, 30-80 parts by weight of the delivery carrier, more than 0 and less than or equal to 10 parts by weight of the solubilizer, 0.05-20 parts by weight of the release-regulating agent, more than 0 and less than or equal to 70 parts by weight of the solvent, and 0-5 parts by weight of the antioxidant; when the drug is selected from the nonsteroidal anti-inflammatory drug, the pharmaceutical composition comprises 0.1-20 parts by weight of the drug, 30-80 parts by weight of the delivery carrier, 0-10 parts by weight of the solubilizer, 0.05-20 parts by weight of the release-regulating agent, more than 0 and less than or equal to 70 parts by weight of the solvent, and 0-5 parts by weight of the antioxidant.

12. A method for preparing the pharmaceutical composition of any one of claims 1 to 11, **characterized by** comprising the step of:
mixing raw material components to obtain the pharmaceutical composition.

13. A pharmaceutical preparation, **characterized by** comprising the pharmaceutical composition of any one of claims 1 to 11.
